# FASCICULE DE BREVET EUROPEEN

(11) **EP 0 819 668 B1**
(45) Date de publication et mention de la délivrance du brevet: **12.12.2001**
(21) Numéro de dépôt: 97401671.9
(22) Date de dépôt: 11.07.1997
(51) Int. Cl.: C07C 17/20, C07C 19/08

(54) **Synthèse du 1,1,1-trifluoroéthane par fluoration du 1-chloro-1,1-difluoroethane**
Synthese von 1,1,1-Trifluorethan durch Fluorierung von 1-Chlor-1,1-Difluorethan
Synthesis of 1,1,1-trifluoroethane by fluorination of 1-chloro-1,1-difluoroethane

(30) Priorité: 16.07.1996 FR 9608874
(43) Date de publication de la demande: 21.01.1998
(73) Titulaire: Atofina, 92800 Puteaux (FR)
(72) Inventeur: Lantz, André, 69390 Vernaison (FR); Perdrieux, Sylvain, 69390 Vernaison (FR); Garrait, Dominique, 69390 Millery (FR); Wendlinger, Laurent, 69230 Saint Genis Laval (FR)
(74) Mandataire: Leboulenger, Jean

(56) Documents cités:
- EP-A- 0 421 830
- EP-A- 0 712 826
- WO-A-96/05156
- CHEMICAL ABSTRACTS, vol. 124, no. 19, 6 mai 1996 Columbus, Ohio, US; abstract no. 260356, GUO X ET AL: "preparation of 1,1,1-trifluoroethane by liquid-phase fluorination of 1,1,1-dichlorofluoroethane" XP002026191 & CN 1 106 779 A (ZHEJIANG CHEM. INST.;PEOP. REP. CHINA) 16 août 1995
- W.B. WHALLEY: "Fluorination of organic compounds with anhydrous hydrogen fluoride. Part I. The preparation of fluoroform and certain derivatives" JOURNAL OF THE SOCIETY OF CHEMICAL INDUSTRY, vol. 66, 1947, LONDON, pages 427-430, XP002026190

## Description

La présente invention concerne le domaine des hydrocarbures fluorés et a plus particulièrement pour objet la fabrication du 1,1,1-trifluoroéthane (connu dans le métier sous la désignation F143a) par fluoration du 1-chloro-1,1-difluoroéthane (F142b) avec de l'acide fluorhydrique anhydre (HF).

Depuis que les chlorofluorocarbures (CFC) ont été identifiés comme l'un des responsables de l'accélération de la dégradation de la couche d'ozone stratosphérique, les acteurs politiques et industriels se sont engagés de manière irréversible dans un processus de substitution des CFC. Ce processus de substitution concerne des secteurs industriels essentiels tels que la chaîne du froid alimentaire, l'isolation des bâtiments, la climatisation, la micro-électronique, etc....

Les recherches pour trouver des remplaçants à ces composés se sont focalisées, dans un premier temps, sur des produits contenant des atomes d'hydrogène (HCFC), puis sur des produits ne contenant plus de chlore : les hydrofluorocarbures (HFC).

L'un de ces composés HFC, ne contenant pas de chlore et donc sans effet sur la couche d'ozone, est le 1,1,1-trifluoroéthane (F143a). Ce composé est principalement destiné, en mélange avec d'autres HFC, à remplacer le F22 (chlorodifluorométhane) et le F502 (mélange azéotropique de F22 et de chloropentafluoroéthane) dans le domaine de la réfrigération, de l'air conditionné et autres applications. L'intérêt est donc important de développer un procédé le plus simple possible pour produire le F143a.

Différents procédés de préparation du F143a ont été décrits dans la littérature. Ainsi, il est connu de préparer le F143a par fluoration du 1,1,1-trichloroéthane soit en phase gazeuse (brevets US 2 744 148 et US 2 744 147), soit en phase liquide. Dans ce dernier cas, la fluoration est de préférence conduite en présence d'un catalyseur de fluoration et la demande de brevet WO 96/5156 préconise en particulier la fluoration du trichloroéthane en présence d'halogénures d'antimoine pentavalent.

La fluoration du fluorure de vinylidène (CF₂ = CH₂) a également été décrite, aussi bien en phase gazeuse (US 2 669 590) qu'en phase liquide (EP 703 204). Ce dernier procédé peut être réalisé en absence de catalyseur et fournirait d'excellents résultats. Cependant, compte-tenu du coût élevé du fluorure de vinylidène, un tel procédé ne paraît pas viable industriellement ; le fluorure de vinylidène est en effet obtenu industriellement par pyrolyse du 1-chloro-1,1-difluoroéthane (F142b) qui est lui-même en général obtenu par fluoration en phase liquide du 1,1,1-trichloroéthane ou du chlorure de vinylidène en l'absence ou en présence de catalyseur (voir par exemple les brevets EP 98341, FR 2 365 542 et EP 421 830).

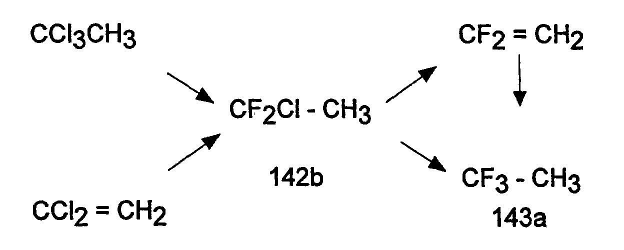

Le F142b est un produit industriel important qui sert comme matière première pour la fabrication du fluorure de vinylidène (VF₂) mais qui, en tant que HCFC, est aussi utilisé en remplacement de certains CFC en particulier comme agent gonflant dans l'industrie des mousses et comme agent propulseur dans l'industrie des aérosols.

Un procédé de fabrication direct et économique de F143a à partir de F142b est donc particulièrement intéressant. II n'existe dans la littérature que deux publications concernant la fluoration du F142b en F143a (brevets US 3 456 025 et US 2 767 227). Ces procédés consistent à réaliser la fluoration du F142b en phase gazeuse à haute température en présence d'un catalyseur de fluoration. Bien que ces deux brevets ne fassent état d'aucun renseignement concernant la durée de vie du catalyseur, on sait que l'inconvénient des procédés de fluoration en phase gaz réside en général dans une rapide désactivation du catalyseur.

Il a maintenant été trouvé qu'en présence d'un catalyseur de fluoration, le F142b et l'acide fluorhydrique anhydre réagissent très rapidement en phase liquide pour former de manière très sélective du F143a.

La présente invention a donc pour objet un procédé de fabrication du F143a, facile à mettre en oeuvre industriellement, caractérisé en ce qu'il comprend la fluoration catalytique en phase liquide du F142b avec de l'acide fluorhydrique anhydre, et en ce qu'il est mis en oeuvre de manière continue dans un réacteur alimenté par du F142b et de l'HF dans un rapport molaire HF/F142b compris entre 1.05 et 1.20.

Le ou les catalyseur(s) de fluoration utilisable(s) dans le procédé selon l'invention sont les catalyseurs actifs bien connus pour les réactions de fluoration en phase liquide tels que les halogénures, oxydes et oxyhalogénures des éléments appartenant aux groupes IIIa, IVa et Va et aux sous-groupes lVb, Vb et Vlb. Parmi les éléments sélectionnés dans les colonnes de la classification périodique, on peut retenir plus spécialement le titane, le niobium, le tantale, le molybdène, le bore, l'étain et l'antimoine. Les espèces contenant de l'antimoine conviennent particulièrement bien. Comme dérivés de l'antimoine, on choisit plus particulièrement les halogénures ; un exemple typique est le pentachlorure d'antimoine SbCl₅ ou les chlorofluorures d'antimoine pentavalent formés in situ par fluoration partielle de SbCl₅.

La quantité de catalyseur à mettre en oeuvre dans cette fluoration en phase liquide peut varier dans de larges limites. Exprimée en pourcentage pondéral de métal, en particulier d'antimoine, la teneur en catalyseur du milieu réactionnel liquide est cependant en général comprise entre 0,01 et 10 %, de préférence entre 0,1 et 5 %.

La possibilité de pouvoir utiliser des quantités aussi faibles de catalyseur est tout-à-fait surprenante car, en général, les fluorations en phase liquide sont réalisées en présence de quantités importantes de catalyseur. Ainsi, dans le procédé de préparation du F143a par fluoration du 1,1,1-trichloroéthane tel que décrit dans la demande de brevet WO 96/5156 précitée, la teneur en catalyseur du milieu réactionnel est de 15 à 50 %.

La pression sous laquelle est mené le procédé selon l'invention n'est pas critique en elle-même, dès lors qu'elle permet d'effectuer la réaction en phase liquide, c'est-à-dire qu'elle est suffisante pour maintenir les réactifs présents dans le réacteur, essentiellement sous forme liquide. Elle varie en fonction de la température du milieu réactionnel et en fonction de la composition de ce milieu réactionnel. La pression absolue du système réactionnel est en général choisie entre 5 et 30 bars, de préférence entre 7 et 20 bars. La réaction de fluoration du F142b en F143a délivre de l'acide chlorhydrique. Si on désire séparer cet HCI par distillation, on a intérêt à réaliser la fluoration sous une pression suffisamment élevée pour pouvoir condenser dans de bonnes conditions l'HCl et, dans ce cas, il est avantageux de travailler à une pression supérieure à environ 11 bars. Ceci n'est cependant pas indispensable et l'HCl peut être séparé par tout autre moyen qu'une distillation, comme par exemple un lavage à l'eau.

Le procédé selon l'invention peut être réalisé dans une large gamme de températures. Généralement, le procédé est mené à une température supérieure à 0°C et inférieure à 120°C. De manière avantageuse, la température ne dépasse cependant pas 100°C, et de préférence, la réaction est conduite à une température comprise entre 10 et 85°C. L'utilisation de températures aussi basses, rendue possible par la très grande réactivité du F142b, permet de minimiser la formation de sous-produits.

En vue de maintenir l'activité du catalyseur, en particulier celle des pentahalogénures d'antimoine, et d'éviter une désactivation par réduction en trihalogénure d'antimoine, on peut avoir intérêt à réaliser la fluoration en présence d'une faible quantité de chlore. Cette addition peut être réalisée de façon périodique ou de façon continue. La quantité du chlore alimenté avec le F142b est en général inférieure à 2,5 moles de chlore pour 100 moles de F142b, de préférence inférieure à 1 % molaire. La quantité de chlore peut être très faible, voire nulle, et elle est d'autant plus faible que la température est plus basse. Il a en effet été montré que, bien que le F142b soit difficile à faire réagir avec le chlore, en présence de SbCl₅ ou d'autres acides de Lewis, il réagit néanmoins pour donner entre autres des produits de chloration de la série 130 (CFCl₂CH₂Cl, CF₂ClCH₂Cl, CCI₂ = CHCl, ...) et surtout de la série 120 (CCl₃CHCl₂, CCl₂FCHCl₂, CF₂ClCHCl₂,...). A basse température, ces réactions de chloration ne sont que très lentes, la consommation de chlore est très faible et il suffit de très faibles quantités de chlore pour maintenir l'activité du catalyseur de fluoration. On utilise donc de préférence entre 0,05 et 0,5 % de chlore (% molaires par rapport au F142b).

Le procédé selon l'invention peut être mis en oeuvre de manière discontinue, mais il est avantageusement effectué de manière continue. Dans ce dernier cas, la réaction peut être réalisée dans un appareillage classique, bien connu de l'homme de l'art. Il peut s'agir d'un réacteur alimenté sous forme gazeuse ou liquide, par les matières premières (F142b et HF) et les recyclages et chauffé ou refroidi de manière adéquate. Il doit favoriser le contact entre les réactifs par une géométrie, un mode d'introduction des réactifs et une technique de mélange appropriés. Le réacteur peut être surmonté d'une colonne et d'un condenseur de rétrogradation permettant d'éviter un départ, dans le flux gazeux issu du réacteur, du ou des catalyseurs utilisés et d'ajuster la composition en organofluorés de ce flux (teneur en F143a, F142b, etc...).

Les matières premières ou recyclats sont alimentés au réacteur dans le rapport adéquat pour une production de F143a. Cela signifie, dans le cas d'un recyclage complet des produits non convertis, un rapport molaire HF frais/F142b frais voisin de la stoechiométrie, c'est-à-dire voisin de 1. Dans la pratique, afin de tenir compte de l'HF éliminé avec le F143a formé, on alimente en général le réacteur avec un mélange HF-F142b correspondant à un rapport molaire HF/142b légèrement supérieur à 1, généralement compris entre 1,05 et 1,20.

Comme indiqué ci-dessus, l'utilisation de chlore pour le maintien de l'activité catalytique se traduit par la formation de produits de chloration (essentiellement des produits de séries 130, 120, 110) ou d'autres sous-produits qui sont des produits lourds par rapport aux F143a et F142b et qui ont donc tendance à s'accumuler dans le réacteur. La concentration du milieu réactionnel en sous-produits lourds n'appartenant pas à la série 140 n'est pas critique, mais il a été constaté que la conduite de la réaction est facilitée lorsque cette teneur n'est pas trop élevée. En règle générale, elle est réglée de manière à rester inférieure à 75 % en poids ; la teneur du milieu réactionnel liquide en sous-produits lourds peut être ajustée par une purge du milieu réactionnel.

Les flux issus de la réaction, gazeux et éventuellement liquides, sont traités de manière conventionnelle pour en séparer les produits finis utiles (F143a, HCI). Pour ce qui concerne la récupération de l'acide chlorhydrique, ce traitement peut en particulier comporter une distillation de l'HCl anhydre ou un lavage à l'eau du flux gazeux afin de séparer l'HCl et les produits organiques (essentiellement F143a). Le F143a, le F142b, les autres produits de la série 140 (F141b, F140a) éventuellement formés, l'acide fluorhydrique non transformé et le ou les catalyseurs utilisés contenus dans la purge liquide du milieu réactionnel peuvent être recyclés au système réactionnel.

Les exemples suivant illustrent l'invention sans la limiter.

### EXEMPLE 1

Dans un réacteur conventionnel de fluoration de 30 litres, en acier ordinaire, surmonté d'une colonne de rétrogradation et d'un condenseur, on a introduit 17 kg de F142b et 209 g de SbCl₅, soit 0,5 % en poids d'antimoine par rapport au F142b. Ce mélange a ensuite été porté à reflux à 70°C, puis on a introduit à cette température :
- 4800 g/h de F142b, soit 47,7 moles/h
- 1020 g/h d'HF, soit 51 moles/h
- 8,5 g/h de Cl₂, soit 0,25 % en moles par rapport au F142b.

La pression a progressivement augmenté et, tout en maintenant la température à 70°C, on l'a laissé monter jusqu'à 17 bars absolus. La pression a ensuite été régulée à cette valeur de 17 bars par soutirage du flux gazeux à la sortie du condenseur de reflux. L'analyse de ce flux gazeux, après lavage à l'eau afin d'éliminer l'HCl produit et l'HF non entièrement transformé, n'a permis de détecter dans le F143a brut qu'une seule impureté organique: le F142b dont la teneur pondérale était voisine de 2000 ppm.

Cet essai a été poursuivi pendant 500 heures au cours desquelles la marche de la réaction (température, pression, débit de sortie, analyse du F143a brut,...) est restée parfaitement stable. Au bout de ces 500 heures de marche, une analyse du milieu réactionnel a permis de constater que ce liquide contenait environ 30 % en poids de produits lourds hors série 140 constitués essentiellement des produits suivants :

| | |
|---|---|
| CCl₃CCl₂H | F120 |
| CFCl₂CCl₂H | F121 |
| CFCl₂CH₂Cl | F131a |
| CF₂ClCH₂Cl | F132b |
| CCl₂ = CHCl | F1120 |

### EXEMPLE 2

Dans le même réacteur que celui de l'exemple 1, on a introduit 17 kg de F142b et 209 g de SbCl₅. Le mélange a ensuite été porté à reflux à 20°C et on a introduit à cette température :
- 5819 g/h de F142b, soit 57,9 moles/h
- 1280 g/h d'HF, soit 64 moles/h
- 20 g/h de Cl₂, soit 0,5 % en moles par rapport au F142b.

On a laissé monter la pression jusqu'à 9 bars absolus, valeur à laquelle elle a été régulée par soutirage du flux gazeux. Après une période de mise en régime, la température de réaction a pu être maintenue entre 15 et 20°C et la marche de la réaction était parfaitement stable. Le F143a brut, obtenu après lavage à l'eau du soutirage gazeux en aval du condenseur, ne contenait qu'une seule impureté détectée : le F142b à une teneur de 2 à 3 % en poids.

La teneur en chlore de ce flux gazeux a été déterminée par analyse, après absorption dans une solution de soude-sulfite de sodium. Aux erreurs d'analyse près, la quantité de chlore trouvée à la sortie correspondait à celle introduite dans le réacteur. Cette très faible réactivité du chlore à cette température a été confirmée par une analyse du milieu réactionnel qui, au bout de 100 heures de marche, contenait moins de 1,5 % de lourds hors série 140.

Après ces 100 heures de marche, on a progressivement laissé monter la température jusqu'à 50°C tout en maintenant la pression à 9 bars. Une analyse du chlore contenu dans le soutirage gazeux a permis de montrer qu'environ 50 % du chlore était consommé. Ceci met bien en évidence l'influence de la température du milieu réactionnel.

## Revendications

1. Procédé de fabrication du 1,1,1-trifluoroéthane (F143a), **caractérisé en ce qu'**il comprend la fluoration catalytique en phase liquide du 1-chloro-1,1-difluoroéthane (F142b) au moyen d'acide fluorhydrique anhydre (HF), et **en ce qu'**il est mis en oeuvre de manière continue dans un réacteur alimenté par du F142b et de l'HF dans un rapport molaire HF/F142b compris entre 1,05 et 1,20.

2. Procédé selon la revendication 1, dans lequel le catalyseur est un halogénure d'antimoine pentavalent, de préférence le pentachlorure d'antimoine ou un chlorofluorure d'antimoine pentavalent.

3. Procédé selon la revendication 2 dans lequel la teneur pondérale en antimoine du milieu réactionnel liquide est comprise entre 0,01 et 10 %, de préférence entre 0,1 et 5 %.

4. Procédé selon l'une des revendications 1 à 3 dans lequel on opère sous une pression absolue comprise entre 5 et 30 bars, de préférence entre 7 et 20 bars.

5. Procédé selon l'une des revendications 1 à 4 dans lequel on opère à une température comprise entre 0 et 120°C, de préférence comprise entre 10 et 85°C.

6. Procédé selon l'une des revendications 1 à 5 dans lequel la réaction est réalisée en présence de chlore, le rapport molaire Cl₂/F142b étant inférieur à 0,025, de préférence inférieur à 0,01 et, plus particulièrement, compris entre 0,0005 et 0,005.

## Patentansprüche

1. , Verfahren zur Herstellung von 1,1,1-Trifluorethan (F143a), **dadurch gekennzeichnet, daß** es die katalytische Fluorierung in flüssiger Phase von 1-Chlor-1,1-difluorethan (F142b) mit wasserfreiem Fluorwasserstoff (HF) enthält und daß es kontinuierlich in einem Reaktor durchgeführt wird, in den F142b und HF in einem Molverhältnis HF/142b zwischen 1,05 und 1,20 eingespeist werden.

2. Verfahren nach Anspruch 1, in dem der Katalysator ein Halogenid von fünfwertigem Antimon, vorzugsweise Antimonpentachlorid oder ein Chlorofluorid von fünfwertigem Antimon, ist.

3. Verfahren nach Anspruch 2, in dem der Gewichtsanteil von Antimon im flüssigen Reaktionsmilieu zwischen 0,01 und 10 %, vorzugsweise zwischen 0,1 und 5 %, liegt.

4. Verfahren nach einem der Ansprüche 1 bis 3, bei dem unter einem Absolutdruck zwischen 5 und 30 bar, vorzugsweise zwischen 7 und 20 bar, gearbeitet wird.

5. Verfahren nach einem der Ansprüche 1 bis 4, **dadurch gekennzeichnet, daß** bei einer Temperatur zwischen 0 und 120 °C, vorzugsweise zwischen 10 und 85 °C, gearbeitet wird.

6. Verfahren nach einem der Ansprüche 1 bis 5, in dem die Reaktion in Gegenwart von Chlor durchgeführt wird, wobei das Molverhältnis Cl₂/F142b unter 0,025, vorzugsweise unter 0,01 und insbesondere zwischen 0,0005 und 0,005, liegt.

## Claims

1. Process for the manufacture of 1,1,1-trifluoroethane (F143a), **characterized in that** it comprises the catalytic fluorination in the liquid phase of 1-chloro-1,1-difluoroethane (F142b) by means of anhydrous hydrofluoric acid (HF), and **in that** it is carried out continuously in a reactor supplied with F142b and HF in an HF/F142b molar ratio of between 1.05 and 1.20.

2. Process according to Claim 1, in which the catalyst is a pentavalent antimony halide, preferably antimony pentachloride, or a pentavalent antimony chlorofluoride.

3. Process according to Claim 2, in which the content by weight of antimony in the liquid reaction mixture is between 0.01 and 10%, preferably between 0.1 and 5%.

4. Process according to one of Claims 1 to 3, in which the operation is carried out under an absolute pressure of between 5 and 30 bar, preferably between 7 and 20 bar.

5. Process according to one of Claims 1 to 4, in which the operation is carried out at a temperature of between 0 and 120°C, preferably of between 10 and 85°C.

6. Process according to one of Claims 1 to 5, in which the reaction is carried out in the presence of chlorine, the Cl₂/F142b molar ratio being less than 0.025, preferably less than 0.01 and, more particularly, between 0.0005 and 0.005.
